# EUROPEAN PATENT APPLICATION

(11) **EP 3 382 844 A1**
(43) Date of publication of application: **03.10.2018**
(21) Application number: 17164383.6
(22) Date of filing: 31.03.2017
(51) Int. Cl.: H02J 7/00, H02J 50/10, A46B 17/06

(54) **WIRELESSLY SUPPLIED TOOL PROCESSING CONTAINER**

(71) Applicant: Koninklijke Philips N.V., 5656 AE Eindhoven (NL)
(72) Inventor: Schroeder, Jessica, 5656 AE Eindhoven (NL)
(74) Representative: Steenbeek, Leonardus Johannes

(57) **Abstract**

A wireless tool processing container, e.g. a brush head dryer (100) includes an inductive charger (103); a base (102) configured to be removably mounted to the inductive charger, the base including a heat conductive material (105); an enclosure (106) arranged to enclose the heat conductive material; a cover (104) configured to form a cavity atop the base and enclose at least one brush head; at least one egress opening (112) to allow heated air to flow from the cover; and at least one ingress opening (110) to allow unheated air to flow into the cover. A wireless method includes: (i) providing the base and a cover; and (ii) causing the heating of the heat conductive material, such that heated air within the cavity can escape through at least one egress opening and unheated air can enter the cavity through at least one ingress opening.

## Description

### Field of the Invention

The present disclosure generally relates to wireless systems and methods for effectively processing tools, such as drying brush heads using induction to transfer power and/or to heat conductive materials.

### Background

Electronic handheld cleaning devices, such as, electric toothbrushes and electric facial cleaning devices have shown to provide superior cleaning results as compared with manual brushing and washing. As with manual toothbrushes, electric toothbrushes are exposed to hundreds of microorganisms found in the oral cavity. Additionally, such electronic devices are exposed to additional microorganisms that are present in the surrounding environment (e.g., a bathroom). Moreover, a brush head can take many hours to dry after use, thus, the presence of moisture accelerates the growth of undesirable mold and bacteria. Although it can be beneficial to store a toothbrush in a container to prevent the brush from picking up surrounding microorganisms, a moist environment in a closed container is more conducive to the growth of microorganisms than the open air. It is desirable to sterilize the brush heads of such electronic devices as often as possible and to prevent or limit the growth of undesirable mold and bacteria.

Several types of systems exist for sterilizing components of electronic devices. For example, conventional systems for sterilizing components of handheld electronic devices include chemicals, either as gases or in liquid form. However, the use of chemical sterilants can be harmful to humans.

Other systems include ultraviolet light irradiation. However, such systems are limited in their effectiveness when it comes to brush heads since the bristles of brush heads are typically configured in close proximity. Shadows can be produced and the entirety of the bristles cannot achieve adequate exposure to the light. Moreover, typical sterilizing systems including ultraviolet-C (UVC) short-wavelength light include sterilizing chambers and such sterilizing chambers occupy valuable countertop space and otherwise limited power outlets.

### Summary of the Invention

Accordingly, there is a need in the art for systems and methods for effectively drying brush heads which do not occupy additional valuable countertop space or require additional power outlets than those that are already in use. The invention is defined by the independent claims. The dependent claims define advantageous embodiments.

The present disclosure is directed to inventive systems and methods for drying tools, e.g. personal care tools such as brush heads wirelessly using induction to heat conductive materials and/or to transfer power to a heating element. The system can include a charger for an electric toothbrush or an electric facial brush such that no additional countertop space or electrical outlets are needed. For example, the base and cover of the inventive systems can be embodied as an accessory that can be coupled with a charger for an electric toothbrush or an electric facial brush. The base of the wireless tool processing container can be configured to cover an inductive charger and the cover of the wireless brush head dryer can be configured to cover the base. The base includes a passive or active heating mechanism. The cover forms a cavity above the base including a volume of air which can be heated by the passive or active heating mechanism. The wireless tool processing container includes openings to allow natural or forced convective airflow. The wireless tool processing container may be arranged to provide other functionality, e.g. cleaning before or during drying e.g. by means of ultraviolet light.

An advantage of one embodiment of the wireless brush head dryer is that the same inductive charger used to charge an electric brush can also be used in connection with the brush head dryer such that no additional countertop space or electrical outlets are needed to use the wireless brush head dryer.

The systems and methods described herein can be configured to hold one or more brush heads. The one or more brush heads includes a neck portion and a brush head portion including bristles, for example, Philips' Sonicare^{®} toothbrush heads which are removable/replaceable relative to the handle portion of the Sonicare^{®} device.

The wireless brush head dryer uses induction to provide heat and/or transfer power. For example, induction is used to heat conductive materials, for example, steel pots and pans. Induction is also used to transfer power between two coils to charge electric devices, for example, handheld electric toothbrushes.

Generally in one aspect, a wireless brush head dryer is provided including: (i) an inductive charger; (ii) a base configured to be removably mounted to the inductive charger, the base including a heat conductive material; (iii) an enclosure arranged to enclose the heat conductive material; (iv) a cover configured to form a cavity atop the base and enclose one or more brush heads; (v) at least one egress opening to allow heated air to flow from the cover; and (vi) at least one ingress opening to allow unheated air to flow into the cover.

The base further may comprise one or more locating features to locate the one or more brush heads and hold the one or more brush heads in a vertical orientation.

The at least one egress opening and the at least one ingress opening may be positioned in the cover. Alternatively, the at least one egress opening may be positioned in the cover and the at least one ingress opening is positioned in the base.

The base may further comprise a printed circuit board assembly.

The base may further comprise an induction receiver coil configured to receive power from an induction transmitter coil.

The base may further comprise an electric heating circuit controlled by the printed circuit board assembly.

The base further may comprise a fan or a pump to cause convection airflow through the cover.

Generally in another aspect, a wireless brush head drying system is provided. The system comprises (i) an inductive charger configured to power and removably couple a plurality of electronic devices including an electronic toothbrush; (ii) a base configured to be removably mounted to the inductive charger, the base including a heat conductive material; (iii) an enclosure arranged to enclose the heat conductive material; (iv) a cover configured to form a cavity atop the base and enclose one or more brush heads; (v) at least one egress opening to allow heated air to flow from the cover; and (vi) at least one ingress opening to allow unheated air to flow into the cover.

Generally in further aspect, a wireless method of drying one or more brush heads is provided. The method comprises the steps of: (i) providing a base comprising an enclosure containing a heat conductive material, and a cover configured to form a cavity atop the base and enclose the one or more brush heads; (ii) receiving, by an induction receiver coil arranged within the base, power from an induction transmitter coil in an inductive charger removably mounted to the base; (iii) selectively activating an electric heating circuit and a convection mechanism within the base to cause the heating of the heat conductive material within the base and convection airflow, wherein heated air within the cavity can escape through at least one egress opening and unheated air can enter the cavity through at least one ingress opening.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### Brief Description of the Drawings

In the drawings, like reference characters generally refer to the same parts throughout the different views. Also, the drawings are not necessarily to scale, emphasis instead generally being placed upon illustrating the principles of the invention.
FIG. 1 is a cross-sectional view of a wireless brush head drying device, in accordance with an embodiment of the present disclosure.
FIG. 2 is a cross-sectional view of a wireless brush head drying device, in accordance with an embodiment of the present disclosure.

### Detailed Description of Embodiments

The present disclosure is directed to wirelessly drying brush heads using a configuration of a base including a conductive heating element and locating features and a cover positioned atop the base. One or more brush heads can be positioned in a vertical, horizontal, or angled orientation by the locating features of the base within an enclosed space within the cover. In operation, the air surrounding the one or more brush head within the cover can be heated by the conductive heating element of the base via induction to dry the one or more brush heads quickly.

A particular non-limiting goal of utilization of the embodiments and implementations herein is to provide effective drying of small objects, such as, brush heads for electric toothbrushes and facial brushes. Examples of power toothbrush devices that can be used with the wireless brush head drying device include Philips' Sonicare^{®} devices. For example, the base of the wireless brush head drying device described herein can be arranged proximate or atop a charging stand of a Sonicare^{®} device such that no additional countertop space or electrical outlets are needed. The base and cover of the inventive systems and methods can be embodied as an accessory that can be coupled with a charger for an electric toothbrush or an electric facial brush.

Advantages of embodiments are illustrated by the following description of exemplary wireless brush head dryers and systems 100, 200. However, the particular components, uses, functionalities and amounts thereof recited in this description, as well as other conditions and details, are to be interpreted to apply broadly in the art and should not be construed to unduly restrict or limit the invention in any way.

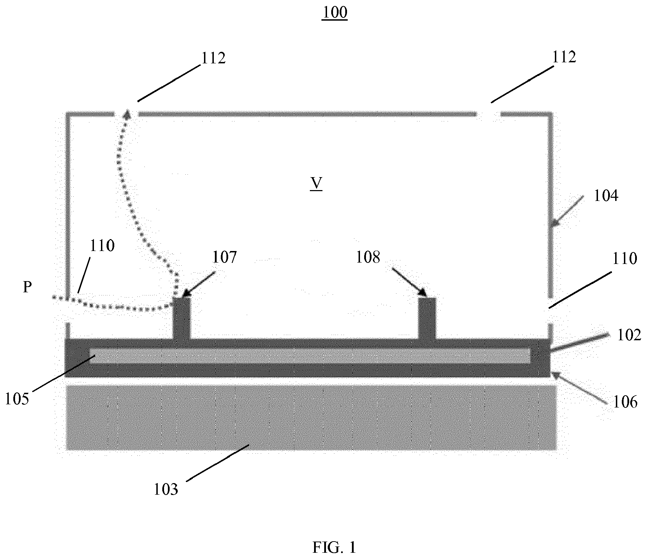

Turning to FIG. 1, a cross-sectional view of a wireless brush head drying device is shown, in accordance with an embodiment. The wireless brush head drying device, shown generally at 100, includes generally a base 102 that fits over an induction charger 103 and a cover 104 that fits on top of the base 102. The base 102 includes a conductive plate 105 that can be heated when exposed to an inductive field. The base 102 further includes an enclosure 106 that prevents a user from contacting the conductive plate 105. The enclosure 106 includes one or more locating features 107, 108 for the brush head(s), the cover 104 and/or the inductive charger 103. The cover 104 encloses a volume of air V to allow the air to become heated above ambient temperature when the conductive plate is providing heat. The cover 104 also provides an airflow path P so that convection can cause air to flow into and out of the enclosed area by ingress and egress openings 110, 112. Any number of ingress and egress openings is contemplated.

The base 102 may be located on top of a transmitter of the inductive charger 103. One or more brush heads (not shown) can be positioned by means of the locating features 107, 108 and the cover 104 can be positioned to cover the brush heads and at least a top surface of the base. The one or more brush heads can be arranged vertically, horizontally or at an angle relative to the base 102 and/or the cover 104. The cover 104 can rest on top of the base 102 or can include snaps or threads for a screw connection.

When the inductive transmitter is powered, the temperature of the conductive plate increases. The air enclosed by the cover is heated by the heated conductive plate. The heated air rises and increases air flow over the one or more brush heads. The warmer air also increases the evaporation rate of the water on the brush head. In operation, the dryer causes the one or more brush heads to dry more quickly.

The locating features 107, 108 can be any suitable features. For example, the locating features 107, 108 can embody protrusions extending upwardly from the enclosure 106 to mate with corresponding indentations in the brush heads. In an example embodiment, the protrusions and indentations can be studs and tubes that are coupled by an interference fit. By way of another example interference fit, the locating features 107, 108 can embody indentations within the enclosure 106 to mate with corresponding protrusions extending downwardly from the brush heads. The protrusions and indentations in either arrangement can be symmetrical or asymmetrical. The locating features 107, 108 in any embodiment can include magnets to strengthen the connection between the feature and the brush head. In another example embodiment, the locating features 107, 108 can include a plurality of soft, flexible silicone extension members. The locating features 107, 108 can also include one or more partial through-bores to receive the entire base of one or more brush heads. Each partial through-bore can include additional locating or retention features, for example, a magnet or some interference fit features. The locating features 107, 108 can include one or more impressions with snap features.

The enclosure 106 may also include features to ensure the cover 104 is removably securable to the base 102 and the base 102 is removably securable to the inductive charger 103. For example, the base 102 can be removably securable to the inductive charger by means of corresponding threading such that the base can include threading on an outer surface or an inner surface to mate with corresponding threading on the charger. The base 102 can be removably securable to the charger by magnets. Alternatively, the base 102 can be removably securable to the charger by a feature that is movable to hold the base 102 in place atop the charger. In an example embodiment, the base 102 and the charger 103 can be correspondingly shaped to be stackable such that when the base 102 is positioned atop the charger 103 the base 102 does not inadvertently become detached from the charger 103.

In an example embodiment, the cover 104 is pivotably connected to the base 102 such that the cover 104 can be pivoted into an open position to allow for the placement of the one or more brush heads. Once positioned, the cover 104 can be pivoted into a closed position for operation. The pivot connection can include one or more pivots. In an example embodiment, the cover can include one or more hinges such that the cover 104 can be selectively opened and closed relative to the base 102.

In the example shown in FIG. 1, the heat conductive plate 105 is inexpensive and embodies a passive form of heating. The plate 105 is passive because it cannot be actively controlled or adjusted. Any suitable metallic conductive material can be used for the plate 105, for example, steel or copper.

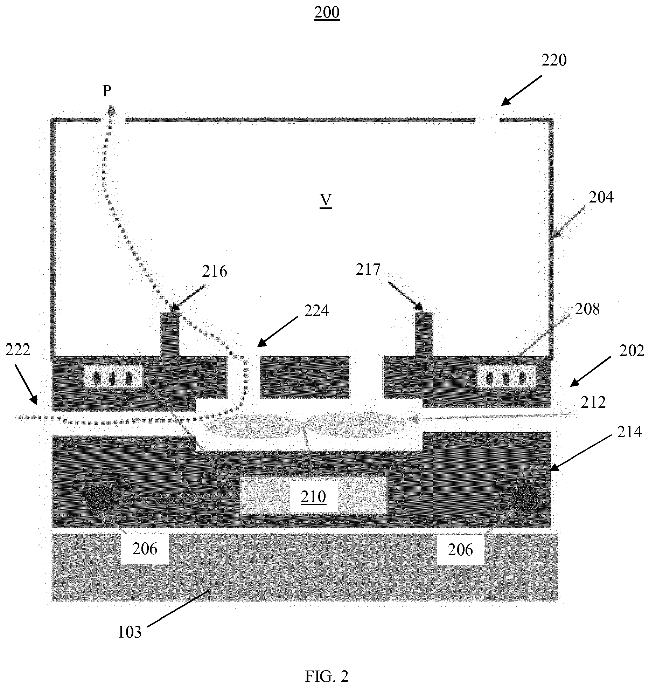

In FIG. 2, a cross-sectional view of a wireless brush head drying device is shown, in accordance with an embodiment. Unlike the wireless brush head drying device shown in FIG. 1 which includes a passive heating element, the wireless brush head drying device in FIG. 2 includes an active heating element which can be controlled and adjusted. The discussion above regarding the wireless brush head drying device 100 applies to the device shown in FIG. 2 except as otherwise described. To allow for additional control or adjustment of the heating and airflow, wireless brush head drying device, shown generally at 200, includes a base 202 that fits over an induction charger 103 and a cover 204 that fits on top of the base 202. The base 202 includes an induction receiver coil 206, an electric heating circuit 208, a printed circuit board assembly 210, a convection mechanism 212, and an enclosure 214. The enclosure 214 supports and protects the induction receiver coil 206, the electric heating circuit 208, the printed circuit board assembly 210, and the convection mechanism 212. Additionally, the enclosure 214 provides locating features 216, 217 for one or more brush heads and the cover 204.

The cover 204 encloses a volume of air V to allow the air to become heated above ambient temperature when the heating circuit is providing heat. The cover 204 also provides an airflow path P so that convection can cause air to flow into and out of the enclosed area via ingress and egress openings 222, 220, respectively. In the embodiment shown in FIG. 2, ingress openings 222,224 are arranged within base 202. One or more ingress openings 222 are arranged in the side of base 202 and one or more ingress openings 224 are arranged within a central portion of the base 202 to allow air to flow from outside into the volume of air V enclosed by the cover 204.

The induction receiver coil 206 receives power from a matching induction transmitter coil in a separate appliance (for example, a charge stand for a Sonicare^{®} device). The induction receiver coil 206 can be tuned through circuitry on the printed circuit board assembly 210 to a specific frequency. The receiver coil 206 and transmitter coil form a transformer. A magnetic field generated by the transmitter coil passes through the receiving coil 206 where it induces an alternating voltage which creates an AC current in the receiver. The induced alternating current may either drive the electric heating circuit 208, the printed circuit board assembly 210, and the convection mechanism 212 directly. Alternatively, the induced alternating current may be rectified to direct current (DC) by a rectifier in the receiver which drives the electric heating circuit 208, the printed circuit board assembly 210, and the convection mechanism 212. The induction receiver coil 206 can be tuned to a 50/60 Hz frequency so AC mains current can be applied directly to the transmitter coil. It should be appreciated that any suitable frequency is contemplated.

The electric heating circuit 208 can be controlled by the printed circuit board assembly 210 to increase the temperature in the space enclosed by the cover 204. In an embodiment, the electric heating circuit 208 is a low voltage electric heating circuit.

The electric heating circuit 208 may include heater wires, for example, Nichrome wire, or any suitable alternative.

The electric heating circuit 208 may include a conductive metal disk or any suitable alternative.

The convection mechanism 212 may be a fan or a pump to cause convection airflow through the space enclosed by the cover 204. The fan or pump can cause the heated air to flow through the space enclosed by the cover 204 more quickly. It is desirable to increase the exchange of air and reduce the drying time of brush heads placed within the enclosed space. The fan or pump can be activated independently with a switch or by the printed circuit board assembly 210. Alternatively, a sensor system can be incorporated to activate the fan or pump only when the one or more brush heads is wet. Still in another example embodiment, the fan or pump can be activated in connection with a timer for a period of time (discussed further below).

The printed circuit board assembly 210 may include circuitry and logic to control the receiver coil 206, the heater coil 208, and the convection mechanism 212. For example, these components can be configured to run a drying cycle which includes a limited temperature range and a limited length of time of heat exposure. The device can further include indicator lights to indicate when the drying cycle is operating and when the cycle has completed. The device can also include moisture sensors to detect moisture on the one or more brush heads.

Light sources can be included within devices 100,200. For example, one or more light sources can include an ultraviolet light source producing a wavelength of approximately 240 nm and can be mounted within the cover 204. Reflective surfaces can also be added within the cover 204 to further ensure the light reaches all areas of a desired brush head to be dried and sterilized. A UV light source can include one or more point sources or a toroidal light source.

Devices 100, 200 can include a timer electrically coupled between a power source and the conductive plate and/or the heater wires and/or the printed circuit board assembly. The timer can be started by a sensor system or switch and can supply power to the conductive plate and/or the heater wires for a predetermined amount of time. After the predetermined amount of time, the power can be disconnected from the conductive plate and/or the heater wires and/or the printed circuit board assembly. The devices 100, 200 can include a display system for indicating how much drying time has elapsed and/or how much drying time is remaining in the drying cycle and/or how many drying cycles have completed. The base and cover of the devices 100, 200 should be made of any suitable materials that do not interfere with the inductance described herein, for example, plastics or woods. Each drying cycle described herein can be for one or more hours. However, a drying cycle can be configured to run for a shorter amount of time. Devices 100, 200 are contemplated for daily use.

The wireless brush head dryer can be part of a system including a universal induction charger configured to power a plurality of electronic devices including an electronic toothbrush or an electronic facial brush.

In a first step of a wireless method for drying one or more brush heads, a base and a cover are provided. The base includes an enclosure containing a heat conductive material. The cover is configured to form a cavity atop the base and enclose one or more brush heads.

In a second step of the method, an induction receiver coil arranged within the base receives power from an induction transmitter coil in an inductive charger removably mounted to the base.

In a third step of the method, an electric heating circuit and a convection mechanism within the base are selectively activated to cause the heating of the heat conductive material within the base and convective airflow. Heated air within the cavity can escape through at least one egress opening and unheated air can enter the cavity through at least one ingress opening. The heat conductive material can be heated passively or actively.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one." Reference signs in the claims and abstract shall not be construed as limiting the scope.

The phrase "and/or," as used herein in the specification and in the claims, should be understood to mean "either or both" of the elements so conjoined, *i.e*., elements that are conjunctively present in some cases and disjunctively present in other cases. Multiple elements listed with "and/or" should be construed in the same fashion, *i.e*., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, *i.e*., the inclusion of at least one, but also including more than one, of a number or list of elements, and, optionally, additional unlisted items. In general, the term "or" as used herein shall only be interpreted as indicating exclusive alternatives (*i.e*., "one or the other but not both") when preceded by terms of exclusivity, such as "either," "one of," "only one of," or "exactly one of"

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "composed of," and the like are to be understood to be open-ended, *i.e*., to mean including but not limited to.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A wireless tool processing container (100, 200), comprising:
a base (102, 202) configured to be removably mounted to an inductive charger (103), the base including a heat conductive material (105, 208);
an enclosure (106, 214) arranged to enclose the heat conductive material;
a cover (104, 204) configured to form a cavity atop the base and enclose a tool;
an egress opening (112, 220) to allow heated air to flow from the cover; and
an ingress opening (110, 222, 224) to allow unheated air to flow into the cover.

2. The wireless tool processing container of claim 1, wherein the base further comprises a locating feature (107, 108, 216, 217) to locate the tool and hold the tool in a vertical orientation.

3. The wireless tool processing container of claim 1 or 2, wherein the egress opening and the ingress opening are positioned in the cover.

4. The wireless tool processing container of claim 1 or 2, wherein the egress opening is positioned in the cover and the ingress opening is positioned in the base.

5. The wireless tool processing container of any of the preceding claims, wherein the base (202) further comprises an induction receiver coil (206) configured to receive power from an induction transmitter coil.

6. The wireless tool processing container of claim 5, wherein the base (202) further comprises an electric heating circuit (208).

7. The wireless tool processing container of claim 5 or 6, wherein the base (202) further comprises a fan (212) to cause convection airflow through the cover.

8. The wireless tool processing container of claim 5 or 6, wherein the base (202) further comprises a pump (212) to cause convection airflow through the cover.

9. A wireless tool processing system (100,200), comprising:
an inductive charger (103) configured to power and removably couple a plurality of electronic devices; and
a wireless tool processing container (100, 200) as claimed in any of the preceding claims.

10. A wireless method of drying a brush head, the method comprising the steps of:
providing a base comprising an enclosure containing a heat conductive material, and a cover configured to form a cavity atop the base and enclose the one or more brush heads;
receiving, by an induction receiver coil arranged within the base, power from an induction transmitter coil in an inductive charger removably mounted to the base;
selectively activating an electric heating circuit and a convection mechanism within the base to cause the heating of the heat conductive material within the base and convection airflow, wherein heated air within the cavity can escape through at least one egress opening and unheated air can enter the cavity through at least one ingress opening.
